(19) Europäisches Patentamt / European Patent Office / Office européen des brevets

(11) **EP 4 763 169 A1**

(12) **EUROPEAN PATENT APPLICATION**

(43) Date of publication:
24.06.2026 Bulletin 2026/26

(21) Application number: 25158149.2

(22) Date of filing: 14.02.2025

(51) International Patent Classification (IPC):
**A61F 13/49** (2006.01)  **B32B 5/02** (2006.01)

(52) Cooperative Patent Classification (CPC):
**A61F 13/49011; A61F 13/4902; B32B 5/022;**
**B32B 5/08; B32B 5/26; B32B 7/05; B32B 27/12;**
B32B 2262/0253; B32B 2262/0276; B32B 2262/12;
B32B 2307/51; B32B 2307/718; B32B 2307/726;
B32B 2555/02

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 23.12.2024 EP 24223115

(71) Applicant: Ontex BV
**9255 Buggenhout (BE)**

(72) Inventors:
• **BUYSSE, Michiel**
**9968 Oosteeklo (BE)**
• **PSCHYKLENK, Lukas**
**56761 Müllenbach (DE)**
• **FRANSEN, Markus**
**56269 Dierdorf (DE)**

(74) Representative: **Macchetta, Andrea**
**Ontex BV**
**Korte Keppestraat 21**
**9320 Erembodegem-Aalst (BE)**

(54) **ABSORBENT ARTICLE COMPRISING A TRI-LAMINATE**

(57) Elastic laminate suitable for use as front and/or back waist belt of a disposable absorbent article comprising a first nonwoven substrate which is mechanically bonded to a second nonwoven substrate. A plurality of elastic strands and/or one or more elastic film(s) is joined in between said second nonwoven substrate and a third nonwoven substrate. The disclosure further provides an absorbent article comprising said elastic laminate and methods of making said absorbent article.

**FIG. 3A**

## Description

### TECHNICAL FIELD

The present disclosure relates to an elastic(s) and nonwoven layered material herein referred to as an elastic nonwoven laminate. The elastic nonwoven laminate may be used in a variety of article and is particularly useful in disposable fluid-handling articles such as baby diapers, adult incontinence articles, feminine hygiene articles, baby swim diapers and any other articles where a cost effective and soft stretchable material may be desirable. The present disclosure also relates to a method and apparatus for manufacturing the elastic nonwoven laminate.

### BACKGROUND

Disposable absorbent articles are often produced on high-speed converting lines using continuous webs of fabrics, films, foams, elastics, etc. Many of these articles preferably include an elastic region or component. Typically this elastic component is covered on at least one side, and preferably two sides, by a nonwoven. This combination of nonwoven-elastic-nonwoven is referred to hereinafter as an elastic nonwoven laminate. The nonwoven portion of the laminate provides softness and opacity when the laminate is under tension so that the skin of a wearer is not visible through it during use. Opacity also provides an indication of the laminates structural integrity.

Elastic nonwoven laminates typically include elastic bonded to a nonwoven. The elastic may include elastic film or elastic strands. In one such laminate, a nonelastic nonwoven is joined to an elastic while the elastic is in a stretched condition so that when the elastic relaxes, the nonwoven gathers between the locations where it is bonded to the elastic forming corrugations. The resulting laminate is stretchable to the extent that the nonwoven gathered between the bond locations allows the elastic to elongate. The process is referred to as live stretch which is further defined herein. U.S. Pat No. 4,720,415 to Vander Wielen, et al., issued January 19, 1988 discloses an example of this type of elastomeric laminate. Since the nonwoven portion of the elastomeric nonwoven laminate is not permanently deformed, the opacity characteristic of the material is not compromised. Therefore, the laminate provides good opacity in a relaxed state or under tension. However, additional nonwoven material is required to compensate for the corrugations.

Elastic strands are typically attached to the nonwoven via an adhesive, which can often results in residual adhesive being exposed between the strands. The exposed adhesive can be problematic for downstream converting operations and for winding the elastomeric nonwoven onto rolls. In addition, exposed elastic strands fail to exhibit the softness and comfort desired by consumers. As a result, elastic nonwoven laminates often include two layers of nonwoven sandwiching the elastic strands. For such embodiments, the stretch properties of the elastic nonwoven laminate may be maintained by attaching the strands to both nonwoven layers simultaneously while under strain forming corrugations in both layers of nonwoven. The dual layers of corrugated nonwoven enhance the opacity characteristics of the laminate; however, further material is required to compensate for the corrugation effect in the second layer of nonwoven.

Another method of producing an elastomeric nonwoven laminate involves "zero strain" stretch laminate webs which comprise at least two plies of material secured to one another along at least a portion of their coextensive surfaces while in a substantially untensioned ("zero strain") condition. One of the plies employed in the "zero strain" stretch laminate is comprised of a material, which is stretchable and elastomeric, i.e., it will return substantially to its untensioned dimensions after an applied tensile force has been released. The second ply secured to the elastomeric ply is elongatable but not necessarily elastomeric. Upon stretching, the second ply will permanently elongate at least to a certain degree, so that upon release of the applied tensile forces, it will not fully return to its original undistorted configuration. The stretching is induced by mechanical activation which includes meshing the laminate between corrugated mating rolls as described in U.S. Patent 5,143,679 issued to Weber, et al. September 1, 1992; U.S. Patent 5,156,793 issued to Buell October 20, 1992; and U.S. Patent 5,167,897 issued to Weber December 1, 1992. Mechanical activation plastically deforms the nonelastomeric nonwoven material reducing the material thickness and creating thin spots in areas where meshing occurs. These thin spots exhibit less opacity, particularly when the laminate is under tension. However, since the nonwoven portion of the laminate is plastically deformed as opposed to being gathered which occurs with live stretch, less material is required in forming the elastomeric laminate.

Consequently, it would be beneficial to provide an elastic nonwoven laminate that uses less elastic, less nonwoven material, less adhesive, or any combination thereof, while having the desired elasticity, opaqueness, and softness desired by the consumer. In particular, it would be beneficial to provide such an elastomeric nonwoven material combining the advantages of live stretch with those of zero strain stretch laminates. It would also be beneficial to provide a method and apparatus for making such a material.

### SUMMARY

In a first aspect of the disclosure an elastic laminate is provided suitable for use as front and/or back waist belt of a disposable absorbent article, preferably a disposable pant, said laminate comprising at least one first nonwoven substrate, at least one second nonwoven substrate; a plurality of elastic strands and/or one or more elastic films, and at least one third nonwoven substrate. Said first nonwoven substrate is mechanically bonded to the second nonwoven substrate at a plurality of discrete bonding elements and the plurality of elastic strands and/or one or more elastic films are sandwiched between the second and third nonwoven substrate. Said first nonwoven substrate comprises a spunbond nonwoven and is different from the second and/or third nonwoven substrate in at least one of basis weight, fiber composition, formation method, and bonding method. Advantageously, an elastic laminate suitable for use as front and/or back waist belt as specified in the first aspect, provides for a softer premium feel in a cost effective way. Said elastic laminate allows to create a soft premium feel without the need to incorporate expensive soft materials.

In a second aspect of the disclosure an absorbent article, preferably a disposable pant, is provided comprising a substantially transversely extending front panel, or waist belt; a substantially transversely extending back panel, or waist belt; and a substantially longitudinally extending absorbent insert joined to each of said front and back panels, or waist belts. Said absorbent insert comprises front and back transversal edges and left and right longitudinal edges connecting the front and back transversal edges to form a perimeter thereof. Furthermore, said absorbent insert comprises an absorbent core and preferably the front and back panels, or waist belts are joined together to define a pair of side seams. Said front and/or back panels, or waist belts, comprise, preferably consist essentially of, even more preferably consist of, an elastic laminate according to the first aspect of the disclosure and its implementation forms according to the present disclosure. Advantageously, an absorbent article comprising front and/or back panels, or waist belts comprising, preferably consisting essentially of, even more preferably consisting of an elastic laminate according to the first aspect of the disclosure and its implementation forms according to the present disclosure, provides for a soft premium feel without the need to incorporate expensive soft materials.

In a third aspect of the disclosure a method of making an absorbent article is provided comprising the steps of providing a first and second nonwoven substrate, stretching the second nonwoven substrate with a greater force compared to the first nonwoven substrate and joining the first nonwoven substrate to the second nonwoven substrate by mechanical bonding at a plurality of discrete bonding elements to form a first elastic laminate precursor. Furthermore, a third nonwoven substrate and a plurality of elastic strands and/or elastic film(s) are provided wherein said first elastic laminate precursor is joined to the third nonwoven substrate with the plurality of elastic strands and/or elastic film(s) therebetween to form an elastic laminate. In said third aspect, an absorbent insert is provided which is joined to the elastic laminate wherein the elastic laminate forms a front and/or back panel, or waist belts, of the absorbent article. The first nonwoven substrate comprises a spunbond nonwoven and the first nonwoven substrate is different from the second and/or third nonwoven substrate in at least one of basis weight, fiber composition, formation method, and bonding method. Advantageously, such method of making an absorbent article allows for cost-effective production of absorbent articles with premium softness.

## BRIEF DESCRIPTION OF THE FIGURES

FIG. 1 is a plan view of a disposable absorbent pant according to embodiments herein.

FIG. 2 is a plan view of a disposable diaper according to embodiments herein.

FIG. 3A is a sectional view taken along the longitudinal axis (y-y) of FIG. 1, showing an elastic laminate according to embodiments herein.

FIG. 3B is a sectional view taken along the longitudinal axis (y-y) of FIG. 1, showing an elastic laminate according to embodiments herein.

FIG. 4 is a sectional view taken along the longitudinal axis (y-y) of FIG. 1 showing the joining the elastic strands and/or one or more films in between the second and third nonwoven substrate according to embodiments herein.

FIG. 5 is a sectional view taken along the longitudinal axis (y-y) of FIG. 1 showing an elastic laminate according to embodiments herein.

FIG. 6 is a sectional view taken along the longitudinal axis (y-y) of FIG. 1 showing a method of making an elastic laminate comprising elastic strands herein.

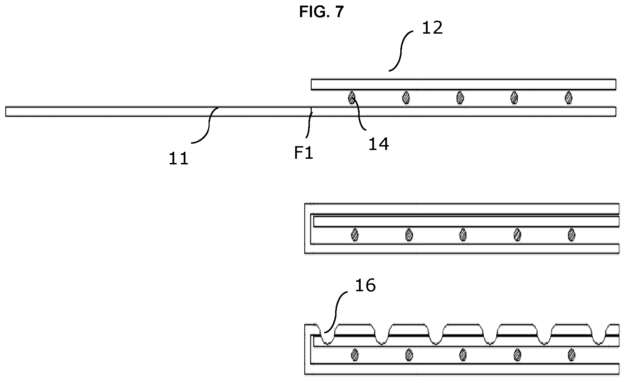

FIG. 7 is a sectional view taken along the longitudinal axis (y-y) of FIG. 1 showing a method of making an elastic laminate comprising elastic strands herein.

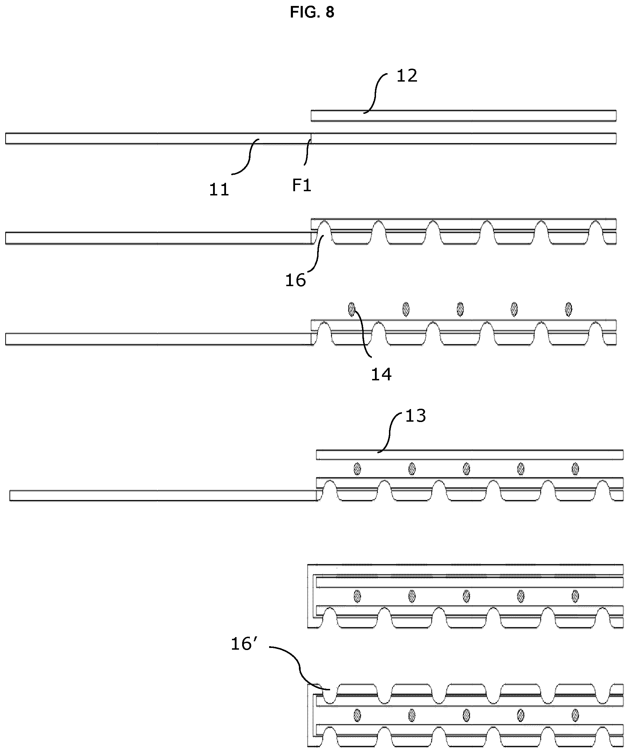

FIG. 8 is a sectional view taken along the longitudinal axis (y-y) of FIG. 1 showing a further embodiment of said method of making an elastic laminate comprising elastic strands herein.

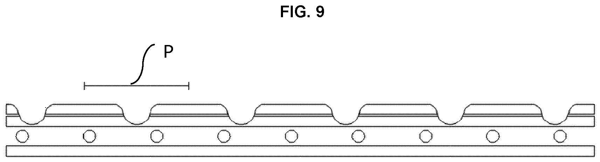

FIG. 9 is a sectional view taken along the longitudinal axis (y-y) of FIG. 1, showing an elastic laminate according to an embodiment herein.

## DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

The expression "% by weight" (weight percent) otherwise referred to as "wt%", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The term "absorbent article" refers to a device that absorbs and contains liquid, and more specifically, refers to a device that is placed against or in proximity to the body of the wearer to absorb and contain the various wastes/exudates discharged from the body. Absorbent articles include but are not limited to diapers, adult incontinence briefs, training pants and diaper holders and liners.

The term "activating" or "activation" refers to the process of making a nonwoven, or an elastic and nonwoven laminate more extensible.

The term "bio-based" may refer to a component of an absorbent article that can be produced or is derived from a renewable resource, as defined herein.

The term "bio-based content" refers to the amount of carbon from a renewable resource in a material as a percent of the mass of the total organic carbon in the material as determined by ASTM D6866-10, method B. In order to apply the methodology of ASTM D6866-10, method B, to determine the bio-based content of any absorbent article or component thereof, a sample may be ground into particulates less than about 20 mesh using known grinding methods (e.g., Wiley® mill (Thomas Scientific, LLC)), and a representative sample of suitable mass taken from the randomly mixed particles. Alternatively, if the sample is merely a layer of material, then the layer itself may be analyzed without the need for a pregrinding step. Determining the bio-based content of a component of an absorbent would first require the component to be dissected, isolated, or cleanly removed from a completed absorbent article as the first step of the analysis. The bio-based content of a component material as described herein would be the mean value of representative sampling of the component as describe above from five different absorbent articles from a given package of absorbent articles or five absorbent articles from randomly purchased packages of a given absorbent article. Note that any carbon from inorganic sources, such as calcium carbonate, is not included in determining the bio-based content of the material. See additional information below for measuring bio-sourced content in polymers. Components of the absorbent articles described in this application may be at least partially comprised of bio-sourced content as described in the following published U.S. patent applications: U.S. Pat. Appl. Pub. Nos. 2007/0219521, 2011/0139658, 2011/0139657, 2011/0152812, 2011/0139662, and 2011/0139659.

The term "Decitex" also known as Dtex is a measurement used in the textile industry for measuring yarns or filaments. 1 Decitex = 1 gram per 10,000 meters. In other words, if 10,000 linear meters of a relaxed yarn or filament weights 500 grams that yarn or filament would have a decitex of 500.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

The terms "elastic," "elastomer," and "elastomeric" may be used herein to refer to any material that is able to extend to a strain of at least 50% without breaking or rupturing when subjected to a tensile force and is able to recover substantially to its original dimensions after the force has been removed.

An "extrusion apparatus" or "extruder" refers herein to any machine capable of extruding a molten stream of material such as a polymeric through one or more extrusion dies.

The term "extrude" or "extruding" refers herein to a process by which a heated elastomer is forced through one or more extrusion dies to form a molten stream of elastic that cools into a solid.

The term "film" refers to a sheet-like material wherein the length and width of the material far exceed the thickness of the material. Typically, films have a thickness of about 0.5 mm or less.

The term "live stretch" includes stretching elastic and bonding the stretched elastic to a nonwoven. After bonding the stretched elastic is released causing it to contract, resulting in a "corrugated" nonwoven. The corrugated nonwoven can stretch as the corrugated portion is pulled to about the point that the nonwoven reaches at least one original flat dimension. The elastic is preferably stretched at least 25% and more preferably at least 100% of its relaxed length when it is bonded to the nonwoven.

The term "molten stream" refers herein to a linear deposit of a heated liquid material such as a polymeric exiting an extrusion apparatus. The stream may include continuous filaments, discontinuous fibers, or continuous films of a polymeric material. When cooled, the molten stream forms a strand elastic.

The term "Natural fibers" refers to elongated substances produced by plants and animals and includes animal-based fibers and plant-based fibers, as those categories are described herein. Natural fibers, as that term is used herein, include fibers harvested without any post-harvest treatment step, as well as those having a post-treatment step, such as, for example, washing, scouring, bleaching, etc.

The term "Naturally-derived" materials include materials that are partially chemically altered without petroleum components and that have been minimally processed such that they not be altered to such an extent that they are substantially less biodegradable or more toxic.

The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern.

The term "Plant-based fibers," as that term is used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

The term "renewable resource" may be used herein to refer to a natural resource that may be produced by a natural process at a rate comparable to its rate of consumption (e.g., within a 100 year time frame). The resource may be replenished naturally, or via engineered agricultural techniques. Non-limiting examples of renewable resources may include plants, animals, fish, bacteria, fungi, and forestry products. These resources may be naturally occurring, hybrids, or genetically engineered organisms. Natural resources such as crude oil, coal, and peat that take longer than 100 years to form are generally not considered to be renewable resources.

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction.

The term "Substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers of fibrous materials, nonwovens, films and foils such as polymeric films or metallic foils. These materials may be used alone or may comprise two or more layers laminated together. As such, a web is a substrate.

GENERAL DESCRIPTION OF AN ABSORBENT ARTICLE

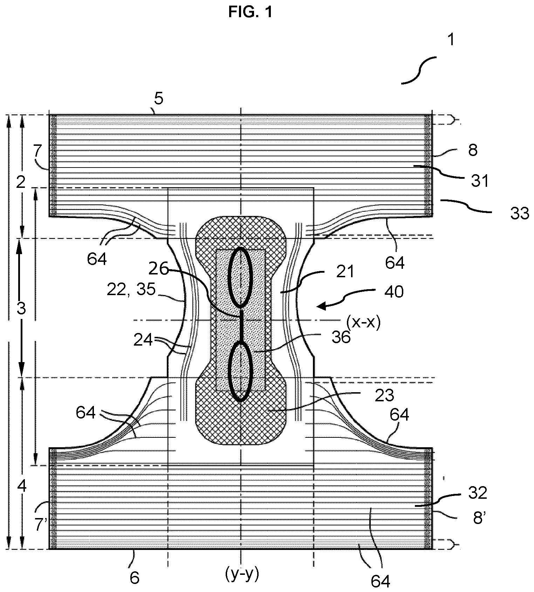

The absorbent article (1) , as exemplified in Fig. 1, may comprise a front waist region (2), a crotch region (3), and a back waist region (4). The crotch region (3) may extend intermediate the front waist region (2) and the back waist region (4). The front waist region (2), the crotch region (3), and the back waist region (4) may each be ⅓ of the length of the absorbent article (1). The absorbent article (1) may comprise a front end edge (5), a back end edge (6) opposite to the front end edge (5), and longitudinally extending, transversely opposed side edges (7 and 8) in the front waist region (2) and longitudinally extending, transversely opposed side edges (7' and 8') in the back waist region (4).

The absorbent article (1) may comprise a liquid permeable topsheet (21), a liquid impermeable backsheet (22), and an absorbent core (23) positioned at least partially intermediate (i.e. between) the topsheet (21) and the backsheet (22). The absorbent article (1) may also comprise one or more pairs of barrier leg cuffs with or without elastics, one or more pairs of outer leg elastics (24), one or more elastic waistbands (25), and/or one or more acquisition materials (26). The acquisition material or materials (26) may be positioned intermediate the topsheet (21) and the absorbent core (23). An outer cover material, such as a nonwoven material, may cover a garment-facing side of the backsheet (22). The absorbent insert (40) may be joined to a wearer-facing surface of the front and back belts (31 and 32) or to a garment-facing surface of the belts via one or more chassis glues or adhesives and/or one or more non-adhesive methods as described herein.

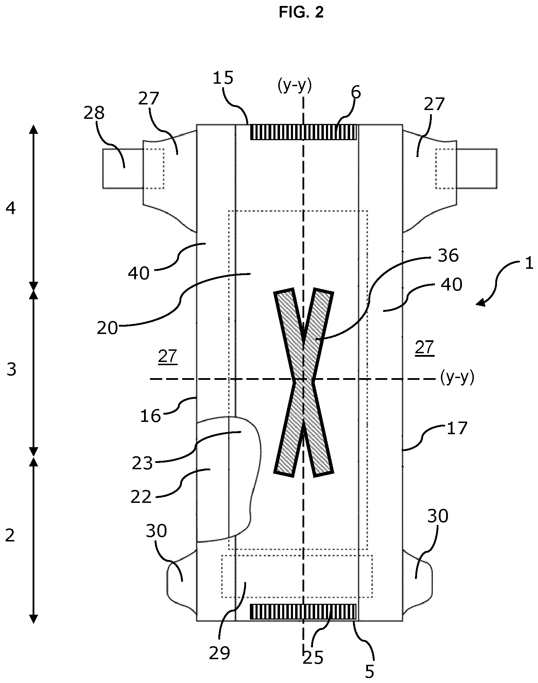

The absorbent article (1), as exemplified in Fig. 2, may comprise back ears (27) in the back waist region (4). The back ears (27) may comprise fasteners (28) and may extend from the back waist region (4) of the absorbent article (1) and attach (using the fasteners (28)) to the landing zone area or landing zone material (29) on a garment-facing portion of the front waist region (2) of the absorbent article (1). The absorbent article (1) may also have front ears (30) in the front waist region (2). The absorbent article (1) may have a central lateral (or transverse) axis (x-x) and a central longitudinal axis (y-y). The central lateral axis (x-x) extends perpendicular to the central longitudinal axis (y-y).

The absorbent article (1) may be in the form of a pant, as exemplified in Fig. 1, having permanent or refastenable side seams. Suitable refastenable seams are disclosed in U.S. Pat. Appl. Pub. No. 2014/0005020 and U.S. Pat. No. 9,421,137.

Side edges of the front belt (7, 8) may be joined to side edges and, respectively, of the back belt (7', 8') to form two side seams (33). The side seams (33) may be any suitable seams known to those of skill in the art, such as butt seams or overlap seams, for example. When the side seams (33) are permanently formed or refastenably closed, the absorbent article (1) in the form of a pant has two leg openings and a waist opening circumference. The side seams (33) may be permanently joined using adhesives or bonds, for example, or may be refastenably closed using hook and loop fasteners (28), for example.

The front and back panels, or front and back belts (31 and 32), may comprise front and back inner belt layers and front and back outer belt layers having an elastic material (e.g., strands (14) and/or one or more film(s) (which may be apertured)) disposed at least partially therebetween. The elastic strands (14) or the one or more film(s) (15) may be relaxed (including being cut) to reduce elastic strain over the absorbent core (23) or, may alternatively, run continuously across the absorbent core (23). The elastics strands (14) and/or elastic films (15) may have uniform or variable spacing therebetween in any portion of the belts (31 and 32). The elastic strands (14) and or the one or more elastic film(s) may also be pre-strained the same amount or different amounts. The front and/or back belts (31 and 32) may have one or more elastic material free zones where the chassis overlaps the belts. In other instances, at least some of the elastic strands (14) and/or one or more of the elastic film(s) (15) may extend continuously across the chassis.

The front and back inner belt layers and the front and back outer belt layers may be joined using adhesives, heat bonds, pressure bonds or thermoplastic bonds. Various suitable belt layer configurations can be found in U.S. Pat. Appl. Pub. No. 2013/0211363.

The front outer belt layer and the back outer belt layer may be separated from each other, such that the layers are discrete or, alternatively, these layers may be continuous, such that a layer runs continuously from the front belt end edge to the back belt end edge. This may also be true for the front and back inner belt layers-that is, they may also be longitudinally discrete or continuous. Further, the front and back outer belt layers may be longitudinally continuous while the front and back inner belt layers are longitudinally discrete, such that a gap is formed between them. Alternatively, instead of attaching belts to the chassis to form a pant, discrete side panels may be attached to side edges of the chassis.

## Top Sheet

The topsheet (21) is typically the outermost layer or component of the absorbent article (1) that is in contact with the wearer's skin. The topsheet (21) may be joined to portions of the backsheet (22), the absorbent core (23), the barrier leg cuffs, and/or any other layers as is known to those of ordinary skill in the art. The topsheet (21) may be compliant, soft-feeling, and non-irritating to the wearer's skin. Further, at least a portion of, or all of, the topsheet (21) may be liquid permeable, permitting liquid bodily exudates to readily penetrate through its thickness. A suitable topsheet (21) may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., a polyester or a polyolefin, such as polyethylene (PE), polypropylene (PP), or bicomponent PE/PP fibers or mixtures thereof), or a combination of natural and synthetic fibers.

In one form of the present disclosure, the topsheet (21) may be a nonwoven material having one or multiple formed layers, nonwovens, or components comprising plant-based fibers other than wood pulp. The nonwoven may be made through well-known techniques; for example, the nonwoven may be a spunbond, a carded and air-through, hydroentangled, or calendar bonded nonwoven. The plant-based fibers may also be spun fibers made at least in part from bio-based materials. For example, the plant-based fibers may be spun poly lactic acid (PLA) fibers or bio-based polyolefin spun fibers. The plant-based fibers may be single component fibers or multi-component fibers, in which less than all of the components are plant-based fibers. One example is a bi-component fiber comprising a polyester core component and a bio-based polyethylene sheath component.

The topsheet (21) may be hydrophilic or hydrophobic or may have hydrophilic and/or hydrophobic portions or layers. If the topsheet (21) is hydrophobic, typically apertures will be present so that bodily exudates may pass through the topsheet (21). The topsheet (21) may have a basis weight of from about 10 gsm to about 40 gsm.

In an embodiment, the topsheet (21) may comprise a bio-based polyolefin with a bio-based content from about 5% to about 100% or from about 10% to about 100% or from about 25% to about 100% or from about 40% to about 100% or from about 50% to about 100% or from about 75% to about 100% or from about 90% to about 100%, using ASTM D6866-10, method B.

## Backsheet

The backsheet (22) is generally that portion of the absorbent article (1) positioned proximate to the garment-facing surface of the absorbent core (23). The backsheet (22) may be joined to portions of the topsheet (21), the outer cover material, the absorbent core (23), and/or any other layers of the absorbent article (1) by any attachment methods known to those of skill in the art. The backsheet (22) prevents, or at least inhibits, the bodily exudates absorbed and contained in the absorbent core (23) from soiling articles such as bedsheets, undergarments, and/or clothing. The backsheet (22) is typically liquid impermeable, or at least substantially liquid impermeable. The backsheet (22) may, for example, be or comprise a thin plastic backsheet film, such as a thermoplastic film having a thickness of about 0.012 mm to about 0.051 mm. Other suitable backsheet (22) materials may include breathable materials or additives as described herein (e.g. calcium carbonate particles) inducing microporous zones in the backsheet film, which permit vapors to escape from the absorbent article, while still preventing, or at least inhibiting, bodily exudates from passing through the backsheet (22). Backsheet films may optionally comprise bio-based materials, such as, for example, bio-based polyethylene, bio-based polypropylene, or other bio-based polyolefins.

In an embodiment, the backsheet (22) may comprise a bio-based polyolefin with a bio-based content from about 5% to about 100% or from about 10% to about 100% or from about 25% to about 100% or from about 40% to about 100% or from about 50% to about 100% or from about 75% to about 100% or from about 90% to about 100%, using ASTM D6866-10, method B.

**Outer Cover Material**

The outer cover material (sometimes referred to as a backsheet nonwoven web) (35) may comprise one or more nonwoven materials joined to the backsheet (22) and that covers the backsheet (22). The outer cover material (35) is typically the layer facing outward-i.e., towards garments or undergarments, when present, and away from the wearer's skin. A caregiver interacts significantly with the outer cover material (35) when holding/comforting the wearer and/or changing the absorbent article (1). The outer cover material (35) forms at least a portion of the garment-facing surface of the absorbent article (1) and effectively "covers" the backsheet (22) so that film is not present on the garment-facing surface. The outer cover material (35) may comprise a bond pattern, apertures, and/or three-dimensional features.

In one form, the outer cover material (35) may be a carded nonwoven or a multi-layered nonwoven comprising carded layers and one or more spunbond layers. The outer cover nonwoven (35) may comprise a combination of plant-based fibers and synthetic fibers that are not plant-based. For example, the nonwoven may comprise both polypropylene fibers and cotton fibers; see, for example, U.S. Pat. Appl. Pub. No. 2017/0203542. The cotton content may range from about 3%, 5%, 10%, or 15% to about 50%, by weight of the nonwoven. When synthetic fibers such as polypropylene are employed, it is preferred that the polypropylene be non-phthalate catalyst polypropylene fibers. Furthermore, the polypropylene fibers may comprise bio-based polyethylene, bio-based polypropylene, or other bio-based polyolefins where such bio-based polyolefin is also phthalate free.

In another form, the outer cover material (35) may comprise a hydroentangled, dual layer nonwoven web, in which one layer comprises a polypropylene spunbond web having a basis weight in the range of from about 5 to 15 gsm and the other layer comprises a carded nonwoven web comprising cotton fibers and a basis weight in the range of from about 10 to about 25 gsm. In a further form, the outer cover material (35) may comprise an air through carded nonwoven web comprising a blend of polypropylene fibers and cotton fibers. The cotton fibers may be included in these nonwoven webs in an amount of about greater than or less than 5%, 10%, or 15% to about 20%, 30%, or 50%, by weight of the overall nonwoven web.

**Absorbent Core**

As used herein, the term "absorbent core" (23) refers to the component of the absorbent article (1) having the most absorbent capacity and that comprises an absorbent material. An absorbent core (23) material may be positioned within a core bag or a core wrap or cover made from, for example, a nonwoven web. The absorbent core (23) material may be profiled or not profiled, depending on the specific absorbent article (1). The absorbent core (23) may comprise, consist essentially of, or consist of, a core wrap, absorbent core (23) material, and core glue or adhesive enclosed within the core wrap. The core wrap may be constructed with one or more nonwoven webs. These nonwoven webs may comprise non-phthalate catalyst polymers. The nonwoven webs may also comprise bio-based materials, including plant-based fibers and/or bio-based materials such as bio-based polyolefins.

The absorbent core (23) material may comprise superabsorbent polymers, a mixture of superabsorbent polymers and air felt (also referred to herein as absorbent pulp), and/or only air felt. The absorbent core (23) material may comprise just the absorbent core (23) materials alone and not include the core wrap, core wrap nonwovens, core glues or adhesives enclosed within the core wrap. The core glue or adhesive may be used to immobilize the superabsorbent polymer particles within the core wrap. In some instances, the absorbent core (23) material may comprise at least 50%, at least 60%, at least 70%, at least 80%, at least 85%, at least 90%, at least 95%, at least 99%, or up to 100% superabsorbent polymers, by weight of the absorbent core (23) material. In some instances, the absorbent core (23) material may be free of air felt, or at least mostly free of air felt. The absorbent core (23) periphery, which may be the periphery of the core wrap, may define any suitable shape, such as rectangular "T," "Y," "hour-glass," or "dog-bone" shaped, for example. An absorbent core (23) periphery having a generally "dog bone" or "hour-glass" shape may taper along its width towards the crotch region (3) of the absorbent article (1).

The absorbent core (23) may have areas having little or no core absorbent material, where a wearer-facing surface of the core wrap may be joined to a garment-facing surface of the core wrap. These areas having little or no core absorbent material may be referred to as "channels" (36). These channels (36) can embody any suitable shapes and any suitable number of channels (36) may be provided. In other instances, the absorbent core (23) may be embossed to create the impression of channels (36).

The superabsorbent polymers of the absorbent core (23) material may comprise a bio-based acrylic acid. In some examples, the superabsorbent polymers may have a bio-based content of from about 5% to about 100%, from about 10% to about 100%, from about 25% to about 100%, from about 40% to about 100%, from about 50% to about 100%, from about 75% to about 100%, or from about 90% to about 100% and all ranges subsumed therein. Superabsorbent polymers comprising a desired bio-based content may be obtained, for example, by mixing acrylic acid derived from conventional (i.e., petroleum-based) sources with bio-based acrylic acid and forming the superabsorbent polymers with the acrylic acid mixture. Superabsorbent polymers comprising the desired bio-based content may also be obtained, for example, by mixing superabsorbent polymers made from conventional acrylic acid with superabsorbent polymers made from bio-based acrylic acid.

Bio-based acrylic acid may be produced using one or more bio-based intermediates. For example, production of conventional, petroleum-based acrylic acid may involve use of petroleum naphtha derived from refining of crude oil to produce propane and/or propene, which may then be oxidized to produce acrylic acid. Bio-based naphtha, which may be derived from renewable materials such plant oils (e.g., palm oil, tall oil, etc.) and animal fat, including waste oils and fats, may be used to produce propane that is then oxidized to produce bio-based acrylic acid. Bio-based propane may be produced from similar renewable sources such as plant and animal waste products and may also occur as a waste product from biofuel production. This bio-based propane may then be oxidized to produce bio-based acrylic acid. In addition, bio-based acrylic acid may be produced (directly) by dehydration of bio-based lactic acid or 3-hydroxy propionic acid, which may be derived from, for example, microbial fermentation of plant-based sources (e.g., corn, rice, waste paper, etc.) and from waste products such as glycerol obtained from biofuel production. These methods may produce acrylic acid of high purity, i.e., a purity of about 98 weight % acrylic acid or higher. While not wishing to be bound by theory, higher purity acrylic acid is critical for achieving high performance superabsorbent polymer characteristics, as well as consumer acceptable performance by the absorbent article (100). Bio-based acrylic acid and methods of production are further described in U.S. Pat. Appl. Pub. No. 2007/0219521 and U.S. Pat. Nos. 8,703,450; 9,630,901; and 9,822,197.

## Acquisition/Distribution Materials

One or more acquisition and/or distribution materials (also referred to herein as "acquisition material(s)") (26) may be present at least partially intermediate the topsheet (21) and the absorbent core (23). The acquisition materials (26) are typically hydrophilic materials that provide significant wicking of bodily exudates. These materials may dewater the topsheet (21) and quickly move bodily exudates into the absorbent core (23). The acquisition materials (26) may comprise one or more nonwoven materials, foams, cellulosic materials, cross-linked cellulosic materials, air laid cellulosic nonwoven materials, spunlace materials, or combinations thereof, for example. The cellulosic materials and cross-linked cellulosic materials may be bleached, but preferably not via chlorine-bleaching so as to be total chlorine free. Hydrogen peroxide is one exemplary bleaching material that is useful in making acquisition materials that are totally chlorine-free. In other embodiments, acquisition and/or distribution materials may comprise non-phthalate catalyst polypropylene fibers and/or plant-based fibers. Furthermore, these nonwoven webs may also comprise bio-based materials, including plant-based fibers and/or bio-based materials such as bio-based polyethylene, bio-based polypropylene, other bio-based polyolefins, bio-based polyesters, and combinations thereof including bi-component fiber configurations.

In some instances, portions of the acquisition materials (26) may extend through portions of the topsheet (21), portions of the topsheet (21) may extend through portions of the acquisition materials (26), and/or the topsheet (21) may be nested with the acquisition materials (26). Typically, an acquisition material (26) may have a width and length that are smaller than the width and length of the topsheet (21). The acquisition material (26) may be a secondary topsheet in the feminine pad context. The acquisition material (26) may have one or more channels as described above with reference to the absorbent core (23) (including the embossed version). The channels in the acquisition material (26) may align or not align with channels (36) in the absorbent core (23). In an example, a first acquisition material may comprise a nonwoven material and as second acquisition material may comprise a cross-linked cellulosic material.

## Barrier Leg Cuffs/Leg Elastics

The absorbent article (1) may comprise one or more pairs of barrier leg cuffs and one or more pairs of outer leg elastics (24). The barrier leg cuffs may be positioned laterally inboard of leg elastics (24). Each barrier leg cuff may be formed by a piece of nonwoven web as described herein that is bonded via a chassis glue or adhesive and/or one or more non-adhesive methods as described herein to the absorbent article (1) so that the barrier leg cuff extends upwards from a wearer-facing surface of the absorbent article (1) and provides improved containment of body exudates approximately at the junction of the torso and legs of the wearer. Useful materials may include, but are not limited to, spunbond-meltblown-spunbond (SMS) nonwoven webs. Such webs may comprise non-phthalate catalyst polypropylene fibers and/or plant-based fibers. Furthermore, these nonwoven webs may also comprise bio-based materials, including plant-based fibers and/or bio-based materials such as bio-based polyolefins.

The barrier leg cuffs are delimited by a proximal edge joined directly or indirectly to the topsheet (21) and/or the backsheet (22) and a free terminal edge, which is intended to contact and form a seal with the wearer's skin. The barrier leg cuffs may extend at least partially between the front end edge (5) and the back end edge (6) of the absorbent article (1) on opposite sides of the central longitudinal axis (y-y) and may be at least present in the crotch region (3). The barrier leg cuffs may each comprise one or more elastics (e.g., elastic strands or strips) near or at the free terminal edge. These elastics cause the barrier leg cuffs to help form a seal around the legs and torso of a wearer. The outer leg elastics (24) extend at least partially between the front end edge (5) and the back end edge (6) of the absorbent article (1). The leg elastics (24) essentially cause portions of the absorbent article (1) proximate to the chassis side edges to help form a seal around the legs of the wearer. The leg elastics (24) may extend at least within the crotch region (3). The leg elastics (24) may be latex-free.

**Elastic Waistband**

The absorbent article (1) may comprise one or more elastic waistbands (25). The elastic waistbands (25) may be positioned on the garment-facing surface or the wearer-facing surface. As an example, a first elastic waistband may be present in the front waist region (2) near the front belt end edge and a second elastic waistband may be present in the back waist region (4) near the back end edge. The elastic waistbands (25) may comprise one or two layers of nonwoven webs with an elastic film or elastic strands therebetween. The elastic waistbands (25) may aid in sealing the absorbent article (1) around a waist of a wearer and at least inhibiting bodily exudates from escaping the absorbent article (1) through the waist opening circumference. In some instances, an elastic waistband (25) may fully surround the waist opening circumference of an absorbent article (1).

**Landing Zone**

The absorbent article (1) may have a landing zone area (29) that is formed in a portion of the garment-facing surface of the outer cover material (35). The landing zone area (29) may be in the back waist region (4) if the absorbent article (1) fastens from front to back or may be in the front waist region (2) if the absorbent article (1) fastens back to front. In some instances, the landing zone (29) may be or may comprise one or more discrete nonwoven materials that are attached to a portion of the outer cover material (35) in the front waist region (2) or the back waist region (4) depending upon whether the absorbent article (1) fastens in the front or the back. In essence, the landing zone (29) is configured to receive the fasteners (28) and may comprise, for example, a plurality of loops configured to be engaged with, a plurality of hooks on the fasteners (28), or vice versa. Landing zone materials, landing zone nonwovens, landing zone nonwovens comprising loops, and fastener hooks may contain polymers containing bio-based content.

**Front and Back Ears**

The absorbent article (1) may have front (30) and/or back ears (27) in a taped diaper context, as exemplified in Fig. 2. Only one set of ears may be required in most taped diapers. The single set of ears, e.g., the back ears, may comprise tape tabs comprising fasteners (also referred to herein as ear hooks) configured to engage the landing zone or landing zone area (29). If two sets of ears are provided, in most instances, only one set of the ears may have fasteners (28), with the other set being free of fasteners (28). The ears, or portions thereof, may be elastic or may have elastic panels. In an example, a stretch or elastic film or stretch or elastic strands may be positioned intermediate a first nonwoven web and a second nonwoven web. In one embodiment, the back set of ears (27) are made from first and second nonwoven webs with an elastic film or elastic strands therebetween, while the front set of ears (30) are made from a generally non-elastic nonwoven web. The nonwoven materials may comprise non-phthalate catalyst polypropylene fibers. Furthermore, these nonwoven materials or webs may also comprise bio-based materials, including plant-based fibers and/or bio-based materials such as bio-based polyolefins.

The elastic film may or may not be apertured. The ears may be shaped. The ears may be integral (e.g., extension of the outer cover material, the backsheet, and/or the topsheet) or may be discrete components attached to a chassis of the absorbent article on a wearer-facing surface, on the garment-facing surface, or intermediate the two surfaces.

**Adhesives**

Adhesives as described herein may be employed to affix one component to another component in the absorbent article's final assembly. Adhesives may also be employed to immobilize sub-components, such as, for example, particulate matter within an absorbent core component. The adhesives in some instances may be devoid of added fluorescence. Furthermore, adhesive may also comprise bio-based materials, including bio-based materials such as bio-based polyolefins, bio-based polyethylenes, bio-based polypropylenes, bio-based alpha olefin polyolefins, and combinations thereof.

## Waist belts / panels

### First preferred embodiment

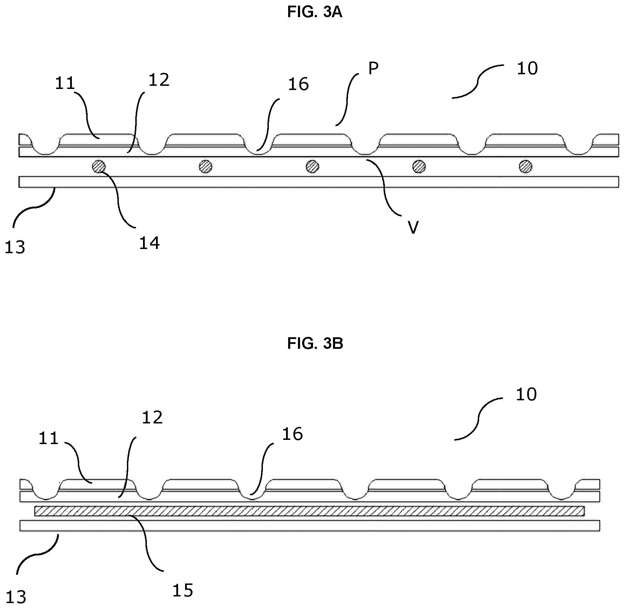

In the first preferred embodiment and as exemplified in Figs. 3A and 3B, an elastic laminate (10) suitable for use as front and/or back waist belt of a disposable absorbent article (1), preferably a disposable pant, comprises at least one first nonwoven substrate (11), at least one second nonwoven substrate (12), a plurality of elastic strands (14) and/or one or more elastic films (15) and at least one third nonwoven substrate (13). Said first nonwoven substrate (11) is mechanically bonded to the second nonwoven substrate (12) at a first plurality of discrete bonding elements (16) and said plurality of elastic strands (14), and/or the one or more elastic films (15), are sandwiched between the second (12) and third nonwoven substrate (13). Furthermore, the first nonwoven substrate (11) comprises a spunbond nonwoven and the first nonwoven substrate (11) is different from the second (12) and/or third nonwoven substrate (13) in at least one of basis weight, fiber composition, formation method, and bonding method. The formation method is selected from the group consisting of drylaid, airlaid, wetlaid, spunlaid, and meltblown; and wherein the bonding method is selected from the group consisting of thermal bonding, mechanical bonding and chemical bonding. Such elastic laminate as disclosed in the present disclosure provides for a softer premium feel in a cost effect way. Said elastic laminate allows to create a soft premium feel without the need to incorporate expensive soft materials.

The elastic laminate (10) according to the first preferred embodiment, comprises elastic strands (14). Alternatively or additionally, said elastic laminate (10) comprises one or more elastic films (15). Advantageously, elastic film material results in a more underwear-like look whereas the use of elastic strands instead of at least a part of the one or more elastic films results in a reduction of plastic content and a more sustainable and eco-friendly product.

The first nonwoven substrate (11) comprises a spunbond nonwoven, preferably consists of a spunbond nonwoven. Typically a spunbond nonwoven is softer than a meltblown nonwoven. Preferably, said first nonwoven substrate (11) comprises a spunmelt nonwoven (SM, SMS). In an example, said first nonwoven substrate (11) comprises a spunbond nonwoven with a basis weight of 16-25 gsm comprising bicomponent fibers, wherein said biocomponent fibers comprise pp/co-pp fibers. The second nonwoven substrate (12) preferably comprises a meltblown nonwoven. Advantageously, by providing a second nonwoven substrate comprising a meltblown nonwoven, the carrier layer for the elastic strands and/or elastic film(s) has greater mechanical performance and tear resistance.

In a first example, according to the first preferred embodiment, said first nonwoven substrate (11) of said elastic laminate (10) corresponds to an outer surface or garment-facing surface and the third nonwoven substrate (13) corresponds to an inner surface or skin-facing surface. Preferably, said spunbond nonwoven of said first nonwoven substrate (11) forms the most outer layer of said elastic laminate (10). The outer surface or garment-facing surface comprises an undulated pattern, as exemplified in Fig. 3A, comprising a plurality of peaks (P) and valleys (V) wherein the valleys (V) correspond to joined areas at the plurality of discrete bonding elements (16) and the peaks (P) correspond to un-joined areas. Advantageously, said undulated pattern comprising a plurality of peaks and valleys provides for a softer outer surface of the article in a cost effective way. Loft surface is therefore created where extra softness is needed without compromising on processability. A softer outer surface provides a softer premium feel to the caregiver who will place the product onto the wearer (e.g. the baby).

In a second example according to the first preferred embodiment and alternative to the first example, the first nonwoven substrate (11) corresponds to an inner surface or skin-facing surface and the third nonwoven substrate (13) corresponds to an outer surface or garment-facing surface. Preferably, said spunbond nonwoven of said first nonwoven substrate (11) forms the most inner layer of said elastic laminate (10) and is in direct contact with the wearer. The inner surface comprises an undulated pattern comprising a plurality of peaks (P) and valleys (V) wherein the valleys (V) correspond to joined areas at the plurality of discrete bonding elements (16) and the peaks (P) correspond to un-joined areas. Advantageously, said undulated pattern comprising a plurality of peaks and valleys provides for a softer inner surface of the article in a cost effective way. Loft surface is therefore created where extra softness is needed without compromising on processability. A softer inner surface provides a softer premium feel to the wearer (e.g. the baby).

In a third example according to any of the first preferred embodiment and previous examples, said second nonwoven substrate (12) comprises a meltblown nonwoven and/or wherein third nonwoven substrate (13) comprises a meltblown nonwoven, preferably said third nonwoven substrate (13) being a multi-layer nonwoven comprising at least one meltblown nonwoven layer and at least one spunbond nonwoven layer wherein the innermost layer of said substrate (13) is a spunbond nonwoven layer when said third nonwoven substrate (13) forms the inner surface of said elastic laminate (10) or wherein the outermost layer of said substrate (13) is a spunbond layer when said third nonwoven substrate (13) forms the outer surface of said elastic laminate (10). Advantageously, greater mechanical performance is provided with some softness by the spunbond layer positioned as the innermost layer of the elastic laminate or the outermost layer of the elastic laminate.

In a fourth example according to any of the first preferred embodiment and previous examples, the elastic laminate (10) comprises elastic strands (14) having an Average-Dtex of greater than 400 dtex, preferably from 450 dtex to 800 dtex, even more preferably from 500 dtex to 700 dtex, even more preferably from 550 dtex to 600 dtex). Alternatively or additionally, said elastic laminate (10) comprises one or more elastic films (15), wherein the elastic film(s) have a basis weight of between about 20 gsm and 100 gsm, preferably between about 25 gsm and 90 gsm, even more preferably between about 30 gsm and 80 gsm, most preferably between about 30 gsm and 60 gsm. Advantageously, elastic film material results in a more underwear-like look whereas the use of elastic strands instead of at least a part of the one or more elastic films results in a reduction of plastic content and a more sustainable and eco-friendly product. Further advantageously, elastic strands having an Average-Dtex of greater than 400 dtex will allow the use of less elastic strands in the product compared to lower dtex elastics thereby reducing plastic content.

Furthermore, said elastic laminate (10) according to said fourth example comprises elastic strands (14) having an Average-Strand-Spacing of more than 4 mm, preferably from 4.5 mm to 12 mm, more preferably from 5 mm to 10 mm, even more preferably from 6 mm to 9 mm. Further advantageously, elastic strands having an Average-Strand-Spacing of more than 4 mm will allow the use of less elastic strands in the product thereby reducing plastic content. Furthermore, elastic strands having a dtex above 400 applied at such Average-Strand-Spacing already results in a texture forming large rugosities which are then further developed by said undulated pattern of peaks and valleys providing additional bulk and lofty feel.

In a fifth example according to any of the first preferred embodiment and examples 1 to 3 and alternatively to the fourth example, said elastic laminate (10) comprises elastic strands (14), the elastic strands (14) having an Average-Dtex from about 10 to about 400 and an Average-Strand-Spacing from about 0.25 mm to about 4 mm. Advantageously, such elastic laminates (10) provide ease of application/donning, improved sustained fit and without marking the wearer's skin.

In a sixth example according to any of the first preferred embodiment and previous examples, the first nonwoven substrate (10) has a first basis weight, the second nonwoven substrate (12) has a second basis weight, and the third nonwoven substrate (13) has a third basis weight, wherein the first basis weight may be greater than the second and/or third basis weight. Preferably, the second basis weight is from 5 gsm to 25 gsm, preferably from 6 gsm to 20 gsm, even more preferably from 7 gsm to 15 gsm; and/or wherein the third basis weight is from 6 gsm to 35 gsm, preferably from 8 gsm to 30 gsm, even more preferably from 10 gsm to 25 gsm. Advantageously, such combination of basis weights allows to create a soft elastic laminate in a cost-effective manner. Providing a higher basis weight at the inner surface and/or outer surface of the elastic laminate compared to the layers between such surfaces, allows to have softer materials in contact with the wearer and/or caregiver while providing lower basis weights in between to have a cost efficient elastic laminate.

Furthermore, said first nonwoven substrate (10) has a first thickness, the second nonwoven substrate (12) has a second thickness, and the third nonwoven substrate (13) has a third thickness, wherein the first thickness may be greater than the second and/or third thickness, preferably the first thickness being at least 1.5 times greater than that of the second thickness. Advantageously, the first nonwoven substrate having a higher thickness provides greater softness/bulkiness where needed, on the outer surface or inner surface of the elastic laminate.

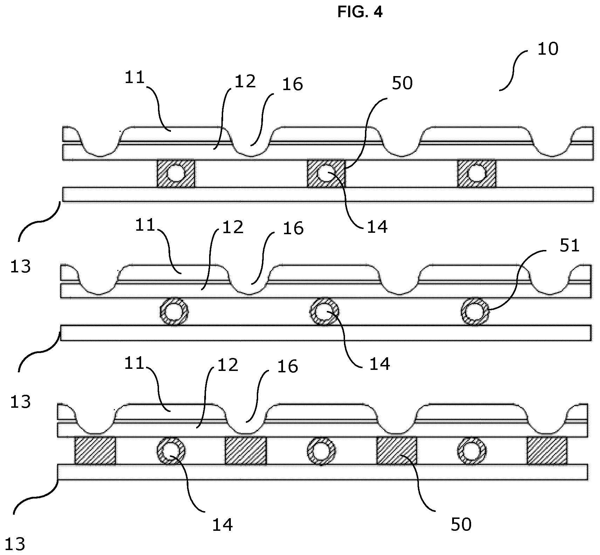

In a seventh example according to the first preferred embodiment and any of the previous examples, as exemplified in Fig. 4, said second nonwoven substrate (12) is joined to the third nonwoven substrate (13) by discontinuous areas of adhesive (50) and/or elastic strands (14) that are strand-coated with adhesive (51), such that one or more areas substantially free of adhesive are formed between the elastic strands. Preferably, said strand-coating being applied in a discrete pattern of strand-coated zones. Additionally or alternatively, the second nonwoven substrate (12) is joined to the third nonwoven substrate (13) by discontinuous areas of adhesive (50) such that one or more areas substantially free of adhesive are formed between the elastic strands. Advantageously, such adhesive application allows to reduce the amount of adhesive being used thereby contributing to a more sustainable and eco-friendly product. Furthermore, reducing the amount of adhesive also has a positive impact on softness of the laminate. This applies to both the outer surface and the inner surface that will come into contact with the skin of the wearer. Furthermore, said laminate has reduced stiffness and improved breathability in view of less phobic substance between skin and outside environment.

In a further implementation of said seventh example, said strand-coated zones of said elastic strands (14) overlap with the first (16) plurality of discrete bonding elements. Advantageously, said undulated pattern comprising a plurality of peaks and valleys is further enhanced and emphasized.

In a further example according to the first preferred embodiment and any of the previous examples, said elastic laminate (10) comprises a plurality of wrinkles and/or repeated pattern of undulations formed by the first nonwoven substrate (11), said plurality of wrinkles and/or repeated pattern of undulations have a pitch extending from one peak to a next neighbouring peak, and said pitch being greater or equal to an Average-Strand-Spacing of the plurality of elastic strands; and preferably said pitch being less than 7 times, preferably less than 6 times, even more preferably less than 5 times, even more preferably less than 4 times, the Average-Strand-Spacing of the plurality of elastic strands. Preferably, said second nonwoven substrate (12) is stretched more than the first nonwoven substrate (11) such that when the second nonwoven substrate (12) is in relaxed state it forms said plurality of wrinkles and/or repeated pattern of undulations resulting in a repeated pattern of peaks and valleys. Advantageously, by applying more stretch to the second nonwoven substrate and the discrete mechanical bonding pattern, an undulated and repeating pattern of peaks and valleys will be formed. Such pattern gives increased softness to the touch by creating a bubble-effect. If the ratio of peak-to-peak is too high, a higher basis weight may be needed to achieve similar softness which would increase cost.

Second preferred embodiment

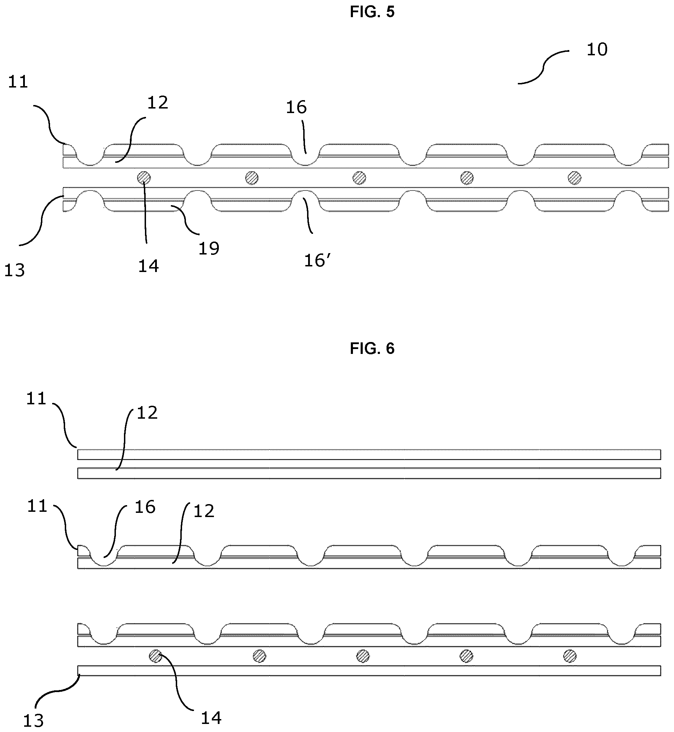

In a second preferred embodiment said elastic laminate (10), according to any of said first preferred embodiment and examples thereof, and as exemplified in Fig. 5, comprises at least one fourth nonwoven substrate (19), wherein said fourth nonwoven substrate (19) is mechanically bonded to the third nonwoven substrate (13) at a second plurality of discrete bonding elements (16'), wherein said fourth nonwoven substrate (19) corresponds to an inner surface or skin-facing surface when said first nonwoven substrate (11) corresponds to an outer surface or garment-facing surface. The inner surface and outer surface comprise an undulated pattern comprising a plurality of peak (P) and valleys (V) wherein the valleys (V) correspond to joined areas at the first and second plurality of discrete bonding elements (16, 16') and the peaks (P) correspond to un-joined areas. Alternatively, said fourth nonwoven substrate (19) corresponds to an outer surface or garment-facing surface when said first nonwoven substrate (11) corresponds to an inner surface or skin-facing surface. The inner surface and outer surface comprise an undulated pattern comprising a plurality of peak (17) and valleys (18) wherein the valleys (18) correspond to joined areas at the first and second plurality of discrete bonding elements (16, 16') and the peaks (P) correspond to un-joined areas. Advantageously, said undulated pattern comprising a plurality of peaks and valleys provides for a softer inner and outer surface of the article in a cost effective way. Loft surface is therefore created where extra softness is needed without compromising on processability. A softer inner and outer surface provides a softer premium feel to the caregiver who will place the product onto the wearer (e.g. the baby) and to the wearer (e.g. the baby).

The fourth nonwoven substrate (19) comprises a spunbond nonwoven, preferably consists of a spunbond nonwoven. Typically a spunbond nonwoven is softer than a meltblown nonwoven. Preferably, said fourth nonwoven substrate (19) comprises a spunmelt nonwoven (SM, SMS). Preferably, said first nonwoven substrate comprises a spunbond nonwoven with a basis weight of 16-25 gsm comprising bicomponent fibers, wherein said biocomponent fibers comprise pp/co-pp fibers. Preferably, said fourth nonwoven substrate (19) is different from the second (12) and/or third nonwoven substrate (13) in at least one of basis weight, fiber composition, formation method, and bonding method.

In a first example according to the second preferred embodiment, said fourth nonwoven substrate (19) has a fourth basis weight which may be greater than the second and/or third basis weight. Advantageously, such combination of basis weights allows to create a soft elastic laminate in a cost-effective manner. Providing a higher basis weight at the inner surface and/or outer surface of the elastic laminate compared to the layers between such surfaces, allows to have softer materials in contact with the wearer and/or caregiver while providing lower basis weights in between to have a cost efficient elastic laminate.

Even further, said fourth nonwoven substrate (19) has a fourth thickness which may be greater than the second and/or third thickness, preferably the fourth thickness being at least 1.5 times greater than that of the second thickness. Advantageously, the first nonwoven substrate and the fourth nonwoven substrate having a higher thickness provides greater softness/bulkiness where needed, on the outer surface and inner surface of the elastic laminate.

In a second example according to any of the second preferred embodiment and first example, said strand-coated zones of said elastic strands (14) overlap with the second (16') plurality of discrete bonding elements. Advantageously, said undulated pattern comprising a plurality of peaks and valleys is further enhanced and emphasized.

In a third example according to any of the second preferred embodiment and examples, said elastic laminate (10) comprises a plurality of wrinkles and/or repeated pattern of undulations formed by the fourth nonwoven substrate (19), said plurality of wrinkles and/or repeated pattern of undulations have a pitch (P) extending from one peak to a next neighbouring peak, and said pitch being greater or equal to an Average-Strand-Spacing of the plurality of elastic strands; and preferably said pitch being less than 7 times, preferably less than 6 times, even more preferably less than 5 times, even more preferably less than 4 times, the Average-Strand-Spacing of the plurality of elastic strands. Advantageously, greater softness can be achieved. If the ratio of peak-to-peak is too high, a higher basis weight may be needed to achieve similar softness which would increase cost.

In a fourth example according to any of the second preferred embodiment and examples, said second nonwoven substrate (12) is coloured or comprises a coloured indicia, and preferably the first nonwoven substrate (11) and/or fourth nonwoven substrate (19) is not coloured, such that the colour can be seen through the first (11) and/or fourth nonwoven substrate (19). Colouring can be done by adding pigments in the fibers or by printing nonwoven substrates. Advantageously, there is a reduced risk of colour migration to the garments or other surfaces because of protection by the first and/or fourth nonwoven substrate. Furthermore, it permits to achieve a premium look in a cost effective way, not only providing extra softness.

Third preferred embodiment

In a third preferred embodiment, an elastic laminate (10) suitable for use as front and/or back waist belt of a disposable absorbent article (1), preferably a disposable pant, comprises at least one first nonwoven substrate (11), at least one second nonwoven substrate (12), a plurality of elastic strands (14) and/or one or more elastic films (15), stretching the second nonwoven substrate (12) with a greater force compared to the first nonwoven substrate (11) and providing a plurality of elastic strands (14) and/or one or more elastic films (15). Said first nonwoven substrate (11) is joined to a first surface of the second nonwoven substrate (12) with the plurality of elastic strands (14) and/or elastic film(s) (15) therebetween to form a first elastic laminate precursor. Said first nonwoven substrate (11) is folded about a first folding axis (F1) thereby overlapping a second surface of the second nonwoven substrate (12) and being bonded thereto by mechanical bonding at a first plurality of discrete bonding elements (16) to form an elastic laminate (10). Advantageously, less operational equipment, e.g. unwinder, is needed. For example, when said first and third nonwoven substrates are not unitary, 2 unwinders would be needed whereas only one is needed when said substrates are unitary.

In a first example according to the third preferred embodiment, said elastic laminate (10) comprises at least one third nonwoven substrate (19), wherein said third nonwoven substrate (13) is joined to a first surface of the second nonwoven substrate (12) with the plurality of elastic strands (14) and/or elastic film(s) (15) therebetween to form a first elastic laminate precursor. Said first nonwoven substrate is bonded to a first surface of said second nonwoven substrate (12) or third nonwoven substrate (13) by a first plurality of discrete bonding elements (16). Said first nonwoven substrate is folded about a first folding axis (F1) thereby overlapping a second surface of said second nonwoven substrate (12) or said third nonwoven substrate (13) and being bonded thereto by mechanical bonding at a second plurality of discrete bonding elements (16') to form an elastic laminate (10). The inner surface and outer surface of said elastic laminate (10) comprise an undulated pattern comprising a plurality of peak (P) and valleys (V) wherein the valleys (V) correspond to joined areas at the first and second plurality of discrete bonding elements (16, 16') and the peaks (P) correspond to un-joined areas. Advantageously, said undulated pattern comprising a plurality of peaks and valleys provides for a softer inner and outer surface of the article in a cost effective way. Loft surface is therefore created where extra softness is needed without compromising on processability. A softer inner and outer surface provides a softer premium feel to the caregiver who will place the product onto the wearer (e.g. the baby) and to the wearer (e.g. the baby). Further advantageously, less operational equipment, e.g. unwinder, is needed. For example, when said first nonwoven substrate (10) would be replaced by 2 separate substrates, 2 unwinders would be necessary instead of 1.

**Absorbent article**

In a first preferred embodiment, an absorbent article (1), preferably a disposable pant, comprises a substantially transversely extending front panel, or waist belt, (31); a substantially transversely extending back panel, or waist belt, (32); and a substantially longitudinally extending absorbent insert (40) joined to each of said front and back panels, or waist belts, (31 and 32), said absorbent insert (40) comprising front and back transversal edges and left and right longitudinal edges connecting the front and back transversal edges to form a perimeter thereof; wherein said insert (40) comprises an absorbent core (23); and preferably wherein the front and back panels, or waist belts, (31 and 32) are joined together to define a pair of side seams (33). The front and/or back panels, or waist belts (31 and 32) comprise, preferably consist essentially of, even more preferably consist of, an elastic laminate (10) according to any of the previous elastic laminate embodiments and examples thereof.

In a first example according to said first preferred absorbent article embodiment, said insert (40) comprises a substantially liquid impermeable backsheet (22) and a substantially liquid permeable topsheet (21) with the absorbent core (23) being sandwiched therebetween; said backsheet (22) comprising one or more first indicia (34) on a garment facing surface thereof, and wherein the second nonwoven substrate (12) of the elastic laminate (10) comprises one or more second indicia (35) on a garment facing surface thereof; and wherein the first and second indicia (34 and 35) are related and/or complement each other. Advantageously, pants are provided with superior softness and optimised cost-performance. Furthermore, more visually appealing pants are provided with continuous/layered appearance permitting a more garment-like feel e.g. jeans look or matching character themes on insert and belt.

**Method of making**

First preferred embodiment

In a first preferred embodiment, as exemplified in Fig. 6, a method of making an absorbent article (1) comprises the steps of providing a first (11) and a second nonwoven substrate (12), stretching the second nonwoven substrate (12) with a greater force compared to the first nonwoven substrate (11) and joining the first nonwoven substrate (11) to the second nonwoven substrate (12) by mechanical bonding at a first plurality of discrete bonding elements (16) to form a first elastic laminate precursor. Further providing a third nonwoven substrate (13) and a plurality of elastic strands (14) and/or one or more elastic films (15). Said first elastic laminate precursor is joined to the third nonwoven substrate (13) with the plurality of elastic strands (14) and/or one or more elastic films (15) therebetween to form an elastic laminate (10). Furthermore, an absorbent insert (22) is provided and joined to the elastic laminate (10) wherein said elastic laminate (10) forms a front and/or back panel, or waist belts, (20 and 21) of the absorbent article (1); and wherein the first nonwoven substrate (11) comprises a spunbond nonwoven and the first nonwoven substrate (11) is different from the second (12) and/or third nonwoven substrate (13) in at least one of basis weight, fiber composition, formation method, and bonding method. Advantageously, such method of making an absorbent article allows for cost-effective production of absorbent articles with premium softness.

In a first example according to the first preferred embodiment of a method of making an absorbent article, said step of joining the elastic strands (14) and/or one or more elastic film(s) (15) between the second nonwoven substrate (12) and the third nonwoven substrate (13) comprises the step of applying discontinuous areas of adhesive and/or elastic strands that are strand-coated with adhesive, such that one or more areas substantially free of adhesive are formed between the elastic strands. Advantageously, greater softness can be achieved by using less adhesive. This applies to both the outer surface and the inner surface that will come into contact with the skin of the wearer.

In a second example, according to said first example, wherein the elastic strands (14) are strand-coated, said strand-coating is applied in a discrete pattern of strand-coated zones. Advantageously, greater softness can be achieved by further reducing adhesive consumption. This applies to both the outer surface and the inner surface that will come into contact with the skin of the wearer. In a further embodiment, said strand-coated zones overlap with the first (16) and/or second plurality (16') of discrete bonding elements. Advantageously, said undulated pattern comprising a plurality of peaks and valleys is further enhanced and emphasized.

In a third example according to any of the first preferred embodiment of a method of making an absorbent article and examples thereof, said first plurality of discrete bonding elements (16) is formed by ultrasonic bonding, preferably applied by a rotating sonotrode. Advantageously, rotating sonotrodes provide high speed repeatability and high stability of the process. Furthermore, ultrasonic bonding results in less contamination compared to glue application patterns.

Second preferred embodiment

In a second preferred embodiment of a method of making an absorbent article (1), according to the first preferred embodiment and examples thereof, a fourth nonwoven substrate (19) is provided and said third nonwoven substrate (13) is stretched with a greater force compared to the fourth nonwoven substrate (19). Said third nonwoven substrate (13) is joined to the fourth nonwoven substrate (19) by mechanical bonding at a second plurality of discrete bonding elements (16') to form a second elastic laminate precursor. In a further step said first elastic laminate precursor is bonded to the second elastic laminate precursor with the plurality of elastic strands (14) and/or elastic films (15) therebetween to form an elastic laminate (10). Advantageously, such method allows to cost-effectively product absorbent articles with premium softness on both the inner surface and outer surface of the waist panels / belts.

In a first example according to the second preferred embodiment of a method of making absorbent articles, prior to the step of joining the first and/or second elastic laminate precursor, said first and/or second elastic laminate precursor is allowed to come to a more relaxed state by reducing the applied tension thereof such that a plurality of wrinkles and/or repeated pattern of undulations are formed by the first and/or fourth nonwoven substrate. Advantageously, by allowing the first and/or second elastic laminate precursor to a more relaxed state by reducing the applied tension thereof to form an undulated and repeating pattern of peaks and valleys. Such pattern gives increased softness to the touch by creating a bubble-effect.

In a second example according to any of the second preferred embodiment of a method of making an absorbent article and the first example, said second plurality of discrete bonding elements (16') is formed by ultrasonic bonding, preferably applied by a rotating sonotrode. Advantageously, rotating sonotrodes provide high speed repeatability and high stability of the process. Furthermore, ultrasonic bonding results in less contamination compared to glue application patterns.

Third preferred embodiment

In a third preferred embodiment, a method of making an absorbent article (1) is provided comprising the steps of providing a first (11) and a second nonwoven substrate (12), stretching the second nonwoven substrate (12) with a greater force compared to the first nonwoven substrate (11) and providing a plurality of elastic strands (14) and/or one or more elastic films (15). Further joining the first nonwoven substrate (11) to a first surface of the second nonwoven substrate (12) with the plurality of elastic strands (14) and/or elastic film(s) (15) therebetween to form a first elastic laminate precursor. In a further step said first nonwoven substrate (11) is folded about a first folding axis (F1) to overlap a second surface of the second nonwoven substrate (12) and said first nonwoven substrate (11) is joined to the second surface of the second nonwoven substrate (12) by mechanical bonding at a first plurality of discrete bonding elements (16) to form an elastic laminate (10). An absorbent insert (40) is provided and joined to the elastic laminate (10), wherein the elastic laminate (10) forms a front and/or back panel, or waist belts, (31 and 32) of the absorbent article (1); and wherein the first nonwoven substrate (11) comprises a spunbond nonwoven and the first nonwoven substrate (11) is different from the second (12) in at least one of basis weight, fiber composition, formation method, and bonding method. Advantageously, such method of making an absorbent article allows for cost-effective production of absorbent articles with premium softness.

In a first example according to said third preferred embodiment of a method of making an absorbent article, a third nonwoven substrate (13) is provided and stretched with a greater force compared to the first nonwoven substrate (11), providing and joining said plurality of elastic strands (14) and/or elastic film(s) (15) between the second nonwoven substrate (12) and third nonwoven substrate (13) to form a first elastic laminate precursor, joining the second nonwoven substrate (12) or the third nonwoven substrate (13) to the first nonwoven substrate (11) by mechanical bonding at a first plurality of discrete bonding elements (16) prior to folding said first nonwoven substrate (11) about said first folding axis (F1) to overlap said second (12) or third nonwoven substrate (13), joining said first nonwoven substrate (11) folded about said first folding axis (F1) to said second (12) or third nonwoven substrate (13) by mechanical bonding at a second plurality of discrete bonding elements (16') to form an elastic laminate (10).

In a second example according to any of said third preferred embodiment and first example of a method of making an absorbent article, the nonwoven substrates are joined by applying elastic strands (14) that are strand-coated in between. Additionally or alternatively, the elastic strands (14) are applied between the nonwoven substrates by applying discontinuous areas of adhesive such that one or more areas substantially free of adhesive are formed between the elastic strands. Advantageously, greater softness can be achieved by using less adhesive. This applies to both the outer surface and the inner surface that will come into contact with the skin of the wearer. Furthermore, said laminate has reduced stiffness and improved breathability in view of less phobic substance between skin and outside environment.

In a third example, according to said second example, wherein the elastic strands (14) are strand-coated, said strand-coating is applied in a discrete pattern of strand-coated zones. Advantageously, greater softness can be achieved by further reducing adhesive consumption. This applies to both the outer surface and the inner surface that will come into contact with the skin of the wearer. In a further embodiment, said strand-coated zones overlap with the first (16) and/or second plurality (16') of discrete bonding elements. Advantageously, said undulated pattern comprising a plurality of peaks and valleys is further enhanced and emphasized.

In a fourth example according to any of said third preferred embodiment and example thereof of a method of making an absorbent article, said first plurality of discrete bonding elements (16) and/or the second plurality of discrete bonding elements (16') are formed by ultrasonic bonding, preferably applied by a rotating sonotrode. Advantageously, rotating sonotrodes provide high speed repeatability and high stability of the process. Furthermore, ultrasonic bonding results in less contamination compared to glue application patterns.

In a fifth example according to any of said third preferred embodiment and examples thereof of a method of making an absorbent article, prior to folding said first nonwoven substrate (11) along said first folding axis (F1), the elastic laminate is allowed to come to a more relaxed state by reducing the applied tension thereof such that a plurality of wrinkles and/or repeated pattern of undulations are formed by the first nonwoven substrate (11). Advantageously, by allowing the first and/or second elastic laminate precursor to a more relaxed state by reducing the applied tension thereof to form an undulated and repeating pattern of peaks and valleys. Such pattern gives increased softness to the touch by creating a bubble-effect.

The embodiments of methods of making absorbent articles according to the present disclosure and their respective embodiments, provide for alternative methods of making an absorbent articles resulting in absorbent articles comprising front and/or back panels, or waist belts with premium soft feel without compromising on operational efficiency and without the need for expensive soft materials. Said methods provide softness in an efficient way by providing a multi-layer elastic laminate in which the difference in stretch applied and the mechanical bonding at a plurality of discrete bonding elements together or alone provide for excellent softness. Therefore, both methods form a single general inventive concept.

## METHODS

### AVERAGE-DECITEX (AVERAGE-DTEX)

The Average-Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastomeric laminates containing elastic fibers is often reported by manufacturers as part of a specification for an elastic fiber or an elastomeric laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g. Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

Calculation of Average-Dtex

For each of the individual elastic fibers f of relaxed length Z, and fiber decitex value $d_i$ (obtained either from the manufacturer's specifications or measured experimentally) present in an absorbent article, or specimen extracted from an absorbent article, the Average-Dtex for that absorbent article or specimen extracted from an absorbent article is defined as:

$$\text{Average-Dtex} = \frac{\sum_{i=1}^{n}(L_i \times d_i)}{\sum_{i=1}^{n} L_i}$$

where n is the total number of elastic fibers present in an absorbent article or specimen extracted from an absorbent article. The Average-Dtex is reported to the nearest integer value of decitex (grams per 10 000 m). If the decitex value of any individual fiber is not known from specifications, it is experimentally determined as described below, and the resulting fiber decitex value(s) are used in the above equation to determine Average-Dtex.

Experimental Determination of Decitex Value for a Fiber

For each of the elastic fibers removed from an absorbent article or specimen extracted from an absorbent article according to the procedure described above, the length of each elastic fiber $L_k$ in its relaxed state is measured and recorded in millimeters (mm) to the nearest mm. Each elastic fiber is analyzed via FT-IR spectroscopy to determine its composition, and its density $\rho_k$ is determined from available literature values. Finally, each fiber is analyzed via SEM. The fiber is cut in three approximately equal locations perpendicularly along its length with a sharp blade to create a clean cross-section for SEM analysis. Three fiber segments with these cross-sections exposed are mounted on an SEM sample holder in a relaxed state, sputter coated with gold, introduced into an SEM for analysis, and imaged at a resolution sufficient to clearly elucidate fiber cross-sections. Fiber cross-sections are oriented as perpendicular as possible to the detector to minimize any oblique distortion in the measured cross-sections. Fiber cross-sections may vary in shape, and some fibers may consist of a plurality of individual filaments. Regardless, the area of each of the three fiber cross-sections is determined (for example, using diameters for round fibers, major and minor axes for elliptical fibers, and image analysis for more complicated shapes), and the average of the three areas $a_k$ for the elastic fiber, in units of micrometers squared ($\mu m^2$), is recorded to the nearest 0.1 $\mu m^2$. The decitex $d_k$ of the kth elastic fiber measured is calculated by:

$$d_k = 10\,000\,\text{m} \times a_k \times \rho_k \times 10^{-6}$$

where $d_k$ is in units of grams (per calculated 10,000 meter length), $a_k$ is in units of $\mu m^2$, and $\rho_k$ is in units of grams per cubic centimeter (g/cm$^3$). For any elastic fiber analyzed, the experimentally determined $L_k$ and $d_k$ values are subsequently used in the expression above for Average-Dtex.

**AVERAGE-STRAND-SPACING**

Using a ruler calibrated against a certified NIST ruler and accurate to 0.5 mm, measure the distance between the two distal strands within a section to the nearest 0.5 mm, and then divide by the number of strands in that section - 1

$$\text{Average-Strand-Spacing} = d/(n-1)$$

where n>1
report to the nearest 0.1 mm.

**Claims**

1. An elastic laminate (10) suitable for use as front and/or back waist belt of a disposable absorbent article (1), preferably a disposable pant, said laminate comprising:

   a. at least one first nonwoven substrate (11);
   b. at least one second nonwoven substrate (12);
   c. a plurality of elastic strands (14) and/or one or more elastic films (15); and
   d. at least one third nonwoven substrate (13);

   wherein the first nonwoven substrate (11) is mechanically bonded to the second nonwoven substrate (12) at a first plurality of discrete bonding elements (16); and **characterized in that** the plurality of elastic strands (14), and/or the one or more elastic films (15), are sandwiched between the second (12) and third nonwoven substrate (13); and **in that** the first nonwoven substrate (11) comprises a spunbond nonwoven and the first nonwoven substrate (11) is different from the second (12) and/or third nonwoven substrate (13) in at least one of basis weight, fiber composition, formation method, and bonding method.

2. An elastic laminate (10) according to Claim 1, wherein the formation method is selected from the group consisting of drylaid, airlaid, wetlaid, spunlaid, and meltblown; and wherein the bonding method is selected from the group consisting of thermal bonding, mechanical bonding and chemical bonding.

3. An elastic laminate (10) according to any of Claims 1 and 2, wherein the first nonwoven substrate (11) corresponds to an outer surface or garment-facing surface and the third nonwoven substrate (13) corresponds to an inner surface or skin-facing surface, and wherein the outer surface comprises an undulated pattern comprising a plurality of peaks (17) and valleys (18) wherein the valleys (18) correspond to joined areas at the plurality of discrete bonding elements (16) and the peaks (17) correspond to unjoined areas; or
   wherein the first nonwoven substrate (11) corresponds to an inner surface or skin-facing surface and the third nonwoven substrate (13) corresponds to an outer surface or garment-facing surface; and wherein the inner surface comprises an undulated pattern comprising a plurality of peaks (17) and valleys (18) wherein the valleys (18) correspond to joined areas at the plurality of discrete bonding elements (16) and the peaks (17) correspond to unjoined areas.

4. An elastic laminate (10) according to any of the preceding Claims, wherein the second nonwoven substrate (12) comprises a meltblown nonwoven and/or wherein the third nonwoven substrate (13) comprises a meltblown nonwoven, preferably said third nonwoven substrate (13) being a multi-layer nonwoven comprising at least one meltblown nonwoven layer and at least one spunbond nonwoven layer wherein the innermost layer of said substrate (13) is a spunbond nonwoven layer when said third nonwoven substrate (13) forms the inner surface of said elastic laminate (10) or wherein the outermost layer of said substrate (13) is a spunbond layer when said third nonwoven substrate (13) forms the outer surface of said elastic laminate (10).

5. An elastic laminate (10) according to any of the preceding Claims, wherein the elastic strands have an Average-Dtex of greater than 400 dtex, preferably from 450 dtex to 800 dtex, even more preferably from 500 dtex to 700 dtex, even more preferably from 550 dtex to 600 dtex;
   and/or wherein the elastic film(s) has a basis weight of between about 20 gsm and 100 gsm, preferably between about 25 gsm and 90 gsm, even more preferably between about 30 gsm and 80 gsm, most preferably between about 30 gsm and 60 gsm.

6. An elastic laminate (10) according to any of the preceding Claims, wherein the first nonwoven substrate (10) has a first basis weight, the second nonwoven substrate having a second basis weight, and the third nonwoven substrate having a third basis weight, wherein the first basis weight is greater than the second and/or third basis weight and wherein the second basis weight is from 5 gsm to 25 gsm, preferably from 6 gsm to 20 gsm, even more preferably from 7 gsm to 15 gsm; and/or wherein the third basis weight is from 6 gsm to 35 gsm, preferably from 8 gsm to 30 gsm, even more preferably from 10 gsm to 25 gsm.

7. An elastic laminate (10) according to any of the preceding Claims, wherein the second nonwoven substrate (12) is joined to the third nonwoven substrate (13) by discontinuous areas of adhesive and/or elastic strands that are strand-coated with adhesive, such that one or more areas substantially free of adhesive are formed between the elastic strands.

8. An elastic laminate (10) according to Claim 7 wherein the elastic strands (14) are strand-coated and wherein the strand-coating is applied in a discrete pattern of strand-coated zones.

9. An elastic laminate (10) according to any of the preceding Claims, wherein the laminate comprises a plurality of wrinkles and/or repeated pattern of undulations formed by the first nonwoven substrate (11), said plurality of wrinkles and/or repeated pattern of undulations have a pitch extending from one peak to a next neighbouring peak, and said pitch being greater or equal to an Average-Strand-Spacing of the plurality of elastic strands; and preferably said pitch being less than 7 times, preferably less than 6 times, even more preferably less than 5 times, even more preferably less than 4 times, the Average-Strand-Spacing of the plurality of elastic strands.

10. An absorbent article (1), preferably a disposable pant, comprising:

- a substantially transversely extending front panel, or waist belt, (31);
- a substantially transversely extending back panel, or waist belt, (32); and
- a substantially longitudinally extending absorbent insert (40) joined to each of said front and back panels, or waist belts, (31 and 32), said absorbent insert (40) comprising front and back transversal edges and left and right longitudinal edges connecting the front and back transversal edges to form a perimeter thereof; wherein said insert (40) comprises an absorbent core (23); and preferably wherein the front and back panels, or waist belts, (31 and 32) are joined together to define a pair of side seams (33);
**characterised in that** the front and/or back panels, or waist belts, (31 and 32) comprise, preferably consist essentially of, even more preferably consist of, an elastic laminate (10) according to any of the preceding claims.

11. An absorbent article (1) according to Claim 10, wherein the insert (40) comprises a substantially liquid impermeable backsheet (22) and a substantially liquid permeable topsheet (21) with the absorbent core (23) being sandwiched therebetween; said backsheet (22) comprising one or more first indicia (34) on a garment facing surface thereof, and wherein the second nonwoven substrate (12) of the elastic laminate (10) comprises one or more second indicia (35) on a garment facing surface thereof; and wherein the first and second indicia (34 and 35) are related and/or complement each other.

12. A method of making an absorbent article (1) comprising the steps of:

a. providing a first nonwoven substrate (11);
b. providing a second nonwoven substrate (12);
c. stretching the second nonwoven substrate (12) with a greater force compared to the first nonwoven substrate (11);
d. joining the first nonwoven substrate (11) to the second nonwoven substrate (12) by mechanical bonding at a first plurality of discrete bonding elements (16) to form a first elastic laminate precursor;
e. providing a third nonwoven substrate (13);
f. providing a plurality of elastic strands (14) and/or one or more elastic films (15);
g. joining the first elastic laminate precursor to the third nonwoven substrate with the plurality of elastic strands (14) and/or elastic films (15) therebetween to form an elastic laminate (10);
h. providing an absorbent insert (22); and
i. joining the absorbent insert (40) to the elastic laminate (10);

wherein the elastic laminate (10) forms a front and/or back panel, or waist belts, (20 and 21) of the absorbent article (1); and wherein the first nonwoven substrate (11) comprises a spunbond nonwoven and the first nonwoven substrate (11) is different from the second (12) and/or third nonwoven substrate (13) in at least one of basis weight, fiber composition, formation method, and bonding method.

13. A method according to Claim 12, wherein the step of joining the elastic strands (14) and/or elastic film(s) (15) between the second nonwoven substrate (12) and the third nonwoven substrate (13) comprises the step of applying discontinuous areas of adhesive and/or elastic strands that are strand-coated with adhesive, such that one or more areas substantially free of adhesive are formed between the elastic strands.

14. A method according to any of Claims 12 to 13 wherein the first plurality of discrete bonding elements (16) are formed by ultrasonic bonding, preferably applied by a rotating sonotrode.

15. A method according to any of Claims 12 to 14 wherein prior to the step of joining the first elastic laminate precursor, said first elastic laminate precursor is allowed to come to a more relaxed state by reducing the applied tension thereof such that a plurality of wrinkles and/or repeated pattern of undulations are formed by the first nonwoven substrate (11).

FIG. 1

**FIG. 2**

FIG. 3A

FIG. 3B

FIG. 4

**FIG. 5**

**FIG. 6**

FIG. 7

**FIG. 8**

FIG. 9

## EUROPEAN SEARCH REPORT

**Application Number**

EP 25 15 8149

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/111616 A1 (MUSLET IYAD [US] ET AL) 14 April 2022 (2022-04-14) * paragraphs [0042] - [0044]; claims 1-20 * ----- | 1-15 | INV. A61F13/49 B32B5/02 |
| A | US 2006/148358 A1 (HALL GREGORY K [US] ET AL) 6 July 2006 (2006-07-06) * paragraphs [0019], [0062] * ----- | 1-15 | |

**TECHNICAL FIELDS SEARCHED (IPC)**

A61F
B32B

The present search report has been drawn up for all claims

| Place of search | Date of completion of the search | Examiner |
|---|---|---|
| Munich | 25 March 2025 | Gennari, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 8149

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2025

| Patent document cited in search report | | Publication date | Patent family member(s) | | Publication date |
|---|---|---|---|---|---|
| US 2022111616 | A1 | 14-04-2022 | CN | 116437884 A | 14-07-2023 |
| | | | JP | 2023545441 A | 30-10-2023 |
| | | | KR | 20230088401 A | 19-06-2023 |
| | | | US | 2022111616 A1 | 14-04-2022 |
| | | | WO | 2022081351 A1 | 21-04-2022 |
| US 2006148358 | A1 | 06-07-2006 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4720415 A, Vander Wielen
- US 5143679 A, Weber
- US 5156793 A, Buell
- US 5167897 A, Weber
- US 20070219521
- US 20110139658 A
- US 20110139657 A
- US 20110152812 A
- US 20110139662 A
- US 20110139659 A
- US 20140005020
- US 9421137 B
- US 20130211363
- US 20170203542
- US 8703450 B
- US 9630901 B
- US 9822197 B